(19) Europäisches Patentamt
European Patent Office
Office européen des brevets

(11) Publication number: **0 407 040 A2**

# EUROPEAN PATENT APPLICATION

(21) Application number: 90306362.6

(22) Date of filing: **12.06.90**

(51) Int. Cl.5: **A61K 7/06, A61K 7/08, C11D 1/62**

(30) Priority: 21.06.89 US 369389
09.04.90 US 507335
21.06.89 US 369361

(43) Date of publication of application:
09.01.91 Bulletin 91/02

(84) Designated Contracting States:
AT BE CH DE DK ES FR GB IT LI LU NL SE

(71) Applicant: **Colgate-Palmolive Company**
**300 Park Avenue**
**New York, N.Y. 10022-7499(US)**

(72) Inventor: **Robbins, Clarence R.**
**1726 Woodfield Road, Martinsville**
**New Jersey(US)**
Inventor: **Dixit, Nagaraj S.**
**3 Titus Lane, Plainsboro**
**New Jersey(US)**
Inventor: **Patel, Amrit M.**
**35 Wetherhill Way, Dayton**
**New Jersey(US)**
Inventor: **Babecki, Raymond E.**
**5 Evergreen Avenue, Fords**
**New Jersey(US)**

(74) Representative: **Kearney, Kevin David**
**Nicholas et al**
**KILBURN & STRODE 30 John Streeteet**
**London, WC1N 2DD(GB)**

(54) Cationic surface active fibre conditioning compositions comprising compounds including long chain hydrocarbyl groups.

(57) A fibre conditioning composition of improved fibre conditioning action includes a cationic surface active fibre conditioning agent and a particular type of water dispersible compound, which contains in its formula a long chain hydrocarbyl group, preferably linear alkyl, of at least 25 carbon atoms, with the proportion of such cationic surface active fibre conditioning agent to long chain hydrocarbyl-containing compound being in the range of 1:20 to 20:1. Because of the coaction of the components of such compositions the deposition of at least one of them on a fibrous substrate is increased, leading to better fibre conditioning action.

Among the described compositions are conditioning rinses and shampoos for human hair, wash cycle and rinse cycle fabric softeners for laundry, and softergents, which are useful for washing laundry and simultaneously softening it.

Preferred water insoluble compounds which contain in their formulas long chain hydrocarbyl grouops of at least 25 carbon atoms include alkanes, carboxylic acids, carboxylic acid esters, amides, amines amine salts, quaternary ammonium salts, and alcohols, some of which are available from certain petrolatums, paraffins, and beeswax.

# CATIONIC SURFACE ACTIVE FIBRE CONDITIONING COMPOSITIONS COMPRISING COMPOUNDS IN-CLUDING LONG CHAIN HYDROCARBYL GROUPS

This invention relates to fibre conditioning compositions. More particularly, it relates to such compositions which comprise a cationic surface active fibre conditioning agent and an essentially water insoluble compound which is water dispersible and has in its formula an exceptionally long chain hydrocarbyl group, which includes at least 25 carbon atoms and preferably is n-alkyl or alkylene. The combination of such a compound and the cationic surface active fibre conditioning agent improves the deposition on to fibrous substrates from aqueous media of at least one of such materials, thereby leading to improved conditioning of such fibers. Also within the invention are a process for manufacturing such effective fiber conditioning compositions and processes for employing such compositions in the forms of conditioning rinses and shampoos for human hair, wash cycle and rinse cycle fabric softeners for laundry, and softergents, which are useful for washing laundry and simultaneously softening it.

Cationic surface active agents (cationic surfactants) have been employed in aqueous compositions for treating fibrous materials, such as human hair and laundry items. In hair treating compositions, such as rinses and shampoos, the cationic surfactants adhere to the hair, even from dilute aqueous media, and help to make it easier to comb and less apt to accumulate electrostatic charges. For laundry the cationic surfactants improve fabric softness and decrease annoying electrostatic charges on fabrics, especially synthetics, which result from drying of the washed laundry in an automatic laundry dryer. Hydrocarbons, such as paraffin waxes, petrolatum, vaseline, and mineral oil, have been employed in hair treating preparations to lubricate the hair, give it body and make it glossy. Both cationic surfactants and hydrocarbons have been applied to fibrous materials from aqueous media and in some cases they have been applied simultaneously with other composition components. However, prior to the present invention the compositions thereof were not made and applied to fibrous materials for applicants' purposes and were not recognized as being of improved conditioning actions due to the presence of the cationic surfactant and the water dispersible compound (WDC) in such compositions.

In accordance with the present invention a fibre conditioning composition comprises a cationic surface active fibre conditioning agent and a water dispersible compound which contains in its formula a long chain hydrocarbyl group of at least 25 carbon atoms (WDC), in which the proportion of cationic surface active fibre conditioning agent to long chain hydrocarbyl-containing compound is in the range of 1:20 to 20:1, in which proportion the presence of both such materials in the composition improves the deposition of at least one of them on a fibrous substrate to be conditioned by application to it of an aqueous medium containing such composition. Among such compositions are conditioning rinses and shampoos for human, wash cycle and rinse cycle fabric softeners for laundry, and softergents for washing laundry and softening it. In some applications the water dispersible compound which contains in its formula a long chain hydrocarbyl group of at least 25 carbon atoms (WDC), acts to promote deposition of the cationic surfactant on to the fibrous strate, even from relatively dilute aqueous media; in other instances the reverse mechanism is observed, in which the cationic surfactant promotes the deposition and adherence of the WDC, and at other times each promotes the deposition and adherence of the other. The final results however are improved conditioning of the fibrous material being treated. Also within the invention are processes for manufacturing the described compositions, and processes in which they are used to condition fibrous substrates.

Searches of the prior art have revealed several patents which may be considered as of relevance to the present invention. Among these the more relevant a considered to be:

U.S. Patents 3,519,562; 3,630,949; 4,184,970; 4,360,437; 4,401,578; 4,426,299; 4,454,049; 4,704,272 and 4,818,523; British patent specification 1,601,360; and Japanese patent 63 08,319.

U.S.Patent 3,519,562 describes textile sewing lubricant compositions containing oxidized Fisher-Tropsch waxes, certain cationic quaternary ammonium salt emulsifiers and water. The oxidized Fisher-Tropsch waxes are different from the preferred WDC's of the present invention, are applied differently to the textiles to be treated, and are applied for a different purpose. U.S. Patent 3,630,949 is for a static dissipating material which is depositable from an aerosol composition that includes a quaternary ammonium chloride and 12-hydroxystearic acid. The compositions of the patent are not in aqueous media and the hydroxystearic acid does not contain the required longer chain alkyl group of applicants' WDC's. U.S. patent 4,184,970 discloses an agglomerate of prills of quaternary ammonium compound intimately mixed with organic dispersion inhibitors and water, wherein the dispersion inhibitor may be a paraffin wax. Although it is taught that octacosane may be employed as a dispersion inhibitor, the compositions of the patents are in particulate form, as opposed to applicants' liquid compositions, which are primarily aqueous, the function of the dispersion inhibitor may not be possible in applicant's compositions and certainly it cannot be prilled with quaternary ammonium compound in applicants' liquid compositions. U.S. Patent 4,360,437 relates to textile treatment compositions for use in the rinse cycles of laundering operations, and such compositions include a cationic fabric softener, a long chain hydrocarbon and specific mono-long chain amines or amine derived compounds. Among the suitable hydrocarbons mentioned in the patent are several that are inoperative for applicants' purpose and the paraffins specifically mentioned are all of shorter alkyl chains than applicants'. U.S. patent 4,401,578 describes concentrated fabric softening compositions containing a cationic fabric softener and a viscosity control agent. The viscosity control agent may be a paraffinic material but those described are all of shorter chains than required for operativeness in the present invention. U.S. patent 4,426,299 includes the same disclosure as U.S. 4,401,578. U.S. Patent 4,454,049 is for rinse cycle textile treatment compositions for fabric softening and static control. Such compositions include cationic fabric softener, water insoluble nonionic extender, which may be a $C_{10-40}$ hydrocarbon, and an organic solvent, with the requirement that the total of such materials is at least 50% of the product. Although the patent mentions the employment of $C_{10-40}$ linear or branched hydrocarbons it appears from the rest of the disclosure that no such materials of 25 or more carbon atoms in an alkyl chain are disclosed. In fact, the patent teaches the successful (for the purpose of the patent) employment of much shorter chain n-paraffins. Also, the patent is for compositions containing substantial proportions of organic solvent, unlike applicants' compositions. U.S.patent 4,704,272 describes shampoos that contain synthetic organic surfactant, water insoluble dispersed silicone, quaternary ammonium salt and suspending agent, such as xanthan gum. The present invention is different because the WDC's thereof are not silicones, and no silicone needs to be present in the compositions of the present invention. U.S. patent 4,818,523 discloses hair rinse conditioners which may include hydrocarbons, such as petrolatum (apparently shorter chain length than $C_{25}$) and mineral oil (which is of branched chain structure) but such materials are not operative in the present invention and do not result in applicants' improved fibre conditioning. British patent specification 1,601,360 is for textile treating compositions comprising certain hydrocarbon materials in combination with specific cationic materials for softening laundry and improving ease of ironing and anti-wrinkling effects. Hydrocarbons of 12 to 40 carbon atoms are mentioned but are not indicated to be preferred and there is no description in the specification of any textile treatment compositions containing applicants' longer chain length WDC's. Japanese patent 63 08,319, according to Chemical Abstracts, 110:121001, is for a hair conditioner containing hydrophobic oils and cationic surfactants. The hydrophobic oils mentioned include liquid paraffin, which does not satisfy the alkyl chain length requirement of applicants' invention.

From the above summary of the closest prior art known to applicants it is seen that their invention is novel and unobvious because there is no effective teaching or suggestion in the prior art of the employment of their longer chain length alkyl-containing WDC's with cationic fibre conditioning surfactant in aqueous media and there is no recognition of the importance of their discovery of the coaction between such components in depositing more fibre conditioning materials than would have been expected, even from relatively dilute aqueous media, on to the fibers to be treated.

The process aspect of the present invention is also novel and unobvious and is important to the obtaining of applicants' unexpectedly beneficial fibre conditioning actions. Similarly, the uses of the different types of fibre conditioning compositions described herein are also novel and unobvious, and result in unexpectedly beneficial conditioning effects.

The cationic fibre conditioning surfactant employed may be any suitable such cationic surfactant which has fabric softening, hair conditioning and/or antistatic properties. Primarily, those cationic materials which are most useful are what will be referred to a quaternary ammonium salts, which are those containing at least one higher molecular weight group and one, two or three lower molecular weight groups, for a total of four, all linked to a common nitrogen atom to produce a cation, and wherein an electrically balancing anion is present, such as a halide, acetate or lower alkosulphate ion, e.g., chloride, bromide or methosulphate. The higher molecular weight substituent (s) on the nitrogen is/are preferably one or more higher alkyl groups, containing 10 or 12 to 22, or 12 to 18 or 20 carbon atoms, such as lauryl, coco-alkyl, myristyl, cetyl, stearyl, tallowalkyl, hydrogenated tallowalkyl or substituted higher alkyl, and the lower molecular weight substituents are preferably lower alkyl of 1 to 3 or 4 carbon atoms, such as methyl or ethyl, or substituted lower alkyl. One or more of said lower molecular weight substituents may include an aryl moiety or may be replaced by aryl, such as benzyl, phenyl or other suitable substituent.

Preferred quaternary ammonium salts for textile treating compositions, such as wash cycle additives, rinse cycle additives and softergents, include di-higher alkyl di-lower alklyl ammonium halides, such as di-tallowalkyl dimethyl ammonium chloride and di- hydrogenated tallowalkyl, dimethyl ammonium chloride and di-hydrogenated tallowalkyl dimethyl ammonium chloride (distearyl dimethyl ammonium chloride, or DSDMAC). Although these same quaternary ammonium salts may be used for hair treating compositions, including rinses, conditioners and shampoos, the prefered quaternary ammonium salts are corresponding mono-higher alkyl, tri-lower alkyl ammonium halides, such as cetyl trimethyl ammonium chloride and bromide, lauryl trimethyl ammonium chloride and stearyl triethyl ammonium chloride. However, it is also possible to employ, at least in part, corresponding tri-higher alkyl mono-lower alkyl ammonium halides, such as trilauryl monoethyl ammonium chloride or tricetyl monomethyl ammonium chloride.

In addition to the cationic compounds previously mentioned, other suitable cationic surfactants include the imidazolinium salts, such as 2-heptadecyl-1-methyl-1-[(2-stearoylamido) ethyl]-imidazolinium chloride; the corresponding methyl sulfate compound; 2-methyl-1-(2-hydroxyethyl)-1-benzyl imidazolinium chloride; 2-coco-1-(2-hydroxyethyl)-1- benzyl imidazolinium chloride; 2-coco-1-(2-hydroxyethyl)-1-octadecenyl imidazolinium chloride; 2-heptadecenyl-1-(2-hydroxyethyl-1-(4-chlorobutyl) imidazolinium chloride; and 2-heptadecyl-1-(hydroxyethyl)-1-octadecyl imidazolinium ethyl sulfate. Generally, the imidazolinium salts of preference will be halides (preferably chlorides) and lower alkyl-sulphates (alkosulphates).

Others of the mentioned quaternary ammonium salts and imidazolinium salts having fabric softening, hair conditioning and antistatic properties may also be employed in the present invention and various others of such compounds are described in U.S. patent 4,000,077, incorporated herein by reference.

Complexes of cationic surfactants with anionic surfactants and with organic acids, such as citric acid, may be substituted, at least in part, for the cationic fiber conditioning agents in the present compositions. Such complexes are described in U.S. patent      , issued on application S.N. 06/916,067, corresponding to GB application No. 8723405, Serial No. 2195653 and in U.S. patent application S.N. 07/189,560, corresponding to .. application No.      , Serial No.      both of which are hereby incorporated by reference.

The water dispersible compound (WDC) of the present compositions, which is so designated for simplicity of identification, is a hydrocarbon, an organic acid, an ester, an amide, an amine, an amine salt, a quaternary ammonium compound or an alcohol, or any mixture thereof, but it is required that each such compound employed in the present compositions, to be effective for the intended purpose, must include in its formula a hydrocarbyl chain of at least 25 carbon atoms. Preferably, such chain is a normal alkyl (or alkylene). It is applicants' discovery that when such a chain is present and is of a length of at least 25 carbon atoms, and sometimes more preferably 25 or 27 to 39 carbon atoms, the fibre treating (conditioning) composition made from it and the cationic surfactant, even in dilution and in the presence of detergent, will very satisfactorily condition fibrous materials, such as hair and laundry, making hair easier to comb and manage, under both wet and dry conditions, and making laundry softer and usually also antistatic. Despite the fact that rinse water and washwater are dilute with respect to the cationic surfactant and the WDC, in these compositions and in these applications the mentioned composition components deposit on the fibers being treated in sufficient amount to have noticeable, desirable and improved conditioning effects. Such results are surprising because the mentioned components coact, producing better conditioning of fibres, rather than causing interference with conditioning, as might have been expected, and such desirable coaction and better deposition of the components on the fibres are obtained in dilute aqueous media. Although some of the WDC and cationic surfactant may remain in the wash water, at least a significant proportion thereof deposits on the fibres (hair or laundry fibres) to condition them, without making them objectionably greasy to the touch. Among the various $C_{25}$ and $C_{25+}$ alkyl-type compounds that are useful as WDC's in practicing the present invention are pentacosane, heptacosane, nonacosane, melene, $C_{31}$ n-alkane, $C_{39}$ n-alkane, cerotic acid, melissic acid, psyllic acid, $C_{26}$ n-alcohol, $C_{28}$ n-alcohol, $C_{36}$ n-alcohol, $C_{25}$ paraffins, $C_{25}$ petrolatums, $C_{33}$ petrolatums, myricyl palmitate, lacceryl palmitate, myricyl cerotate, myricyl hypogaete, ceryl-2-hydroxypalmitate, mono- and diamines and amine salts of the mentioned hydrocarbons, alcohols of the mentioned hydro-carbons and amides of the mentioned acids. As will be seen, the various mentioned WDC's all include at least one normal alkyl or alkylene group of from 25 - 39 carbon atoms, which may be considered as a more preferred range. However, the mentioned compounds are listed as representative and not exclusive. Also while saturated compounds are preferred, usually, in part, at least, for stability reasons, unsaturated materials are also useful.

The various WDC's may be employed as pure compounds or in mixtures. They may ne synthetic or may be obtained from natural materials. Some have been systhesized and may be employed as the WDC's in the present compositions, or they may be mixed with other such systhesized compounds. Alternatively such compounds may be obtained from natural materials and products of chemical refineries, such as oil refeineries. Such materials and products may be processed to produce higher concentrations of the more desirable WDC's or they may be employed directly. Among some sources of WDC's which are components of the compositions of the present invention are petrolatums, paraffin waxes, beeswax, various waxes from the group of candellilla, Japan, bayberry and montan, and other natural waxes which include the requisite amounts of $C_{25}$ and $C_{25+}$ normal alkyl-or normal alkylene-containing compounds. The purified components of beeswax include nonacosane, melissic acid, myricyl cerotate, and n-hentriacontanyl trimethyl ammonium

4

chloride, nonacosyl amine and melissamide, may be considered as derivatives of such purified components. Experimentation has shown that carnauba wax does not produce the desired results but microcrystalline waxes, which include secondary branched chains and primary alicyclic chains, in addition to normal alkane chains, have been found to be useful, which apparently indicates that some cyclization and branching in an alkyl chain do not seriously interfere with the fiber conditioning actions of the $C_{25}$ and longer alkyl- and alkylene-containing compounds. Accordingly, branching like that of microcrystalline waxes is within the n-alkane description of the WDC's of this invention. Of course, providing that there was a normal $C_{25}$ chain, branching and the presence of other groups would not cause compounds to be outside the description of the WDC's of the invention.

Paraffins that may also be utilized will normally be of chain lengths of 20 to 50 carbon atoms, preferably 20 to 40 carbon atoms. Isoparaffins that can be used are preferably of chain lengths in the range of 12 to 16 carbon atoms, prefreably 13 to 14 carbon atoms. The petrolatums are desirably petroleum jellies or mineral jellies which melt in the range of 38 to 60 °C. The microcrystalline waxes are preferably of an average molecular weight in the range of about 500 to 800 (which is about twice that of the paraffins). $C_{18-36}$ fatty acids and corresponding triglycerides are higher fatty acids and triglycerides which are available from Croda Chemical Corporation (under the tradename Syncrowax HGL-C, for example, for the triglycerides). Stearyl stearate, which is representative of useful esters of both higher fatty alcohols and higher fatty acids, is available from Inolex Corporation, as Lexol SS.

In addition to the cationic surfactant and WDC components of the present composition the only other required component of the conditioning rinse for the hair and of the wash cycle and rinse cycle compositions for laundry is an aqueous medium, in which the cationic surfactant and the WDC are emulsified or otherwise dispersed. When in the form of an emulsion, with hydrotrope, emulsifying agent and/or other surfactant desirably being present, to be of good stability and to be most effective in conditioning fibrous materials the composition should be made by a certain procedure, which will be described subsequently. The aqueous medium may be water or an aqueous alcoholic medium and may also contain various normal adjuvants, such as are employed in compositions of the types described. The water utilized is preferably deionized water but tap waters are also satisfactory. The alcohol employed, if any will normally be denatured alcohol, such as SD-40, and when it is present in the fiber conditioning compositions it will usually constitute 3 to 15% of the final composition, e.g. about 5%.

The synthetic organic detergent component of the present shampoo and softergent compositions is a synthetic organic anionic detergent or a synthetic organic nonionic detergent, or a mixture thereof (and of course, mixtures of anionic detergents and mixtures of nonionic detergents may also be employed). Optionally, amphoteric, ampholytic, zwitterionic and even cationic detergents and surface active agents (surfactants), in addition to the cationic fibre conditioning surfactants, may be present in the compositions of the present invention. Various suitable detergents that may be employed are listed in McCutcheon's Detergents and Emulsifiers ,North American Edition, 1984, which is incorporated by reference herein. Of those compounds the preferred anionic detergents are of the sulphated and/or sulphonated types, which may be designated as "sulph(on)ated". Such detergents are water soluble salts of a lipophile sulfuric or sulfonic acid. The lipophilic moiety of such acid will normally be of 8 to 30 carbon atoms and will desirably include an alkyl group, preferably a chain, of 8 to 22 or 10 to 18 or 20 carbon atoms, more preferably 10 to 14 or 16 carbon atoms, e.g. about 12 carbon atoms. Among such anionic detergents there may be mentioned, as exemplary thereof, the higher alkyl sulfates, the linear higher alkylbenzene sulfonates, the paraffin sulfonates, olefin sulfonates, monoglyceride sulfates and higher fatty alcohol lower alkoxy sulfates. Such higher fatty alcohol or higher alkyl lower alkoxy sulfates are preferably of 10 to 16 carbon atoms in the higher fatty alcohol or alkyl moiety thereof and of up to 20 moles of lower alkoxy per mole, such as 1 to 20, more preferably 2 to 4 or 2 to 6, e.g., 2 or 3 (with the lower alkoxy normally being ethoxy but sometimes also including up to 30% propoxy), and such are preferred detergents for shampoos. A particularly preferred higher fatty alcohol sulfphate is lauryl sulfate and a particularly prefered higher fatty alcohol poly-lower alkoxy sulfate is that wherein the higher fatty alcohol is lauryl alcohol, which is triethoxylated. For softergents the more preferred anionic detergents are linear higher alkylbenzene sulfonates, such as linear dodecylbenzene sulfonate. For the shampoos the anionic detergents are usually ammonium and/or triethanolamine salts or mixtures of one or more thereof and sodium salts, and for the softergents the salts are normally of sodium, such as the sodium salts of the above named detergents.

Although the invented detergent compositions are preferably anionic, they may also be nonionic or primarily nonionic, in which case the major nonionic detergent component will normally be a lower alkoxylated alcohol or phenol (the phenol may be substituted with an alkyl group, usually a chain of 8 to 10 carbon atoms). The lower alkoxy is normally ethoxy but may also be at least partially propoxy (up to 30% of the alkoxy). Preferably the nonionic detergent will be a condensation product of a higher fatty alcohol of 8 to 15 carbon atoms with 5 to 12 moles of lower alkylene oxide, more preferably with the higher fatty alcohol being of 8 to 12 carbon atoms and condensed with 6 to 10 moles of lower alkylene oxide. Similarly, when the ethylene oxide is condensed with alkyl phenol, the moles of ethylene oxide employed will be in similar ranges per mole of phenol as per mole of alcohol. For both the ethoxylated alkyl phenols it is prefered that they be of the narrow range ethoxylate type (NRE's), but B(broad)RE's are also useful.

Although shampoo and softergent compositions can be made which utilize only nonionic detergent as the detersive surfactant it is often preferable to incorporate some anionic detergent in such compositions because nonionic detergents are normally low foaming and it is usually desirable to have the detergent composition capable of producing foam, especially for shampooing. While any of the anionic detergents previously mentioned in the specification may be employed in supplementation of the nonionic detergent, when a nonionic detergent is present, it has been found preferable to utilize one which is of a sulphate or sulfonate type, such as a fatty alkyl sulphate of 8 to 18 or 10 to 16 carbon atoms, e.g., ammonium or triethanolammonium lauryl sulfate (TEALS). Various adjuvants may be utilized together with the requuired components of the compositions of the present invention for the properties which they impart. In all the liquid compositions of the invention ethanol and/or other normally polar co-solvent may be included, and usually colorants and perfumes will also be incorporated, with foam modifying agents, opacifying agents, thickening agents and pearlescing agents being optional. For hair conditioning compositions, such as rinses, there may also be present higher fatty alcohols, such as cetyl alcohol, which has a thickening effect, thickeners, such as hydroxyethyl cellulose, and neutralizing agents and buffers, such as sodium borate. For fabric softening wash cycle additives and rinse additives for treatment of laundry it has been found that small proportions of sequestering salts, such as sodium citrate, and lower alkylene glycols, such as propylene glycols, help to improve the rheology of the liquid product, acting as thinners. For conditioning shampoos, the adjuvants may include thickeners and opacifiers, such as cetyl alcohol, stearyl stearate, and ethylene glycol distearate, other thickeners, such as xanthan gum, Natrosol (Registered Trademark) (hydroxyethyl cellulose), Methocel (Registered Trademark) (methyl cellulose), and sodium chloride, preservatives, antioxidants, sequestrants, and lower alkylene glycol solubilizers and thinners, such as propylene glycol. For the softergents additional useful adjuvants include fluorescent brighteners, such as distilbene brighteners, preservatives, such as formalin, and neutralizing agents, including triethanolamine. In addition to those adjuvants mentioned, others known in the art for employment with liquid compositions of the types of this invention may also be present and when particulate solid compositions are made appropriate components of such compositions may also be utilized, including flow promoting agents, anti-redeposition agents, fabric softening powders, such as bentonite, builders, such as polyacetal carboxylate, zeolite, sodium tripolyphosphate and sodium carbonate, bleaches, such as sodium perborate, bleach activators and/or stabilizers, and soil release promoting agents, such as polyethylene terephthalate - polyoxyethylene terephthalate copolymers (PET-POET copolymers).

The various proportions of required components in the described compositions are important for obtaining the improved properties for the compositions that have been described herein. For all the compositions of the present invention the proportion of cationic surface active fibre conditioning agent to WDC should be such that the presences of both such materials in the compositions improve the deposition of at least one of them on to a fibrous substrate to be conditioned by application to it of an aqueous medium containing the composition of the present invention. It has been found that such proportion of the two mentioned components is normally in the range 1:20 to 20:1, preferably being in the range of 1:3 to 20:1 and 0.5:1 to 20:1, respectively. Percentagewise, the aqueous emulsion compositions of the invention, which are preferably based on quaternary ammonium salt as the cationic surface active fiber conditioning agent, will normally include 0.1 to 20% of such quaternary ammonium salt and 0.1 to 10% of the WDC, which is often preferably a n-alkane.

For conditioning rinses for human hair, usually for employment after shampooing, the percentages of quaternary ammonium salt, WDC and aqueous medium will normally be in the ranges of 0.2 to 10%, 0.1 to 5% and 75 to 99%, respectively, preferably being 0.2 to 5%, 0.1 to 2% and 85 to 98%, more preferably 0.3 to 2%, 0.1 to 15% and 90 to 97%, and most preferably 0.4 to 1.3%, 0.1 to 0.5% and 95 to 97%. The balances, if any, will be adjuvants, which are also the balances of the other compositions within the invention which are described subsequently herein. In the described compositions the preferred aqueous medium is water, the more preferred quaternary ammonium halide is higher fatty alkyl tri-lower alkyl ammonium halide wherein the higher alkyl is of 12 to 18 carbon atoms and lower alkyls are of 1 to 3 carbon atoms, and the WDC is selected from the group consisting of alkanes, carboxylic acids, carboxylic acid esters and alcohols of alkyl chains of at least 25 carbon atoms, and mixtures thereof. For the most preferred compositions the quaternary ammonium halides are cetyl trimethyl ammonium chloride, dicetyl dimethyl ammonium chloride and distearyl dimethyl ammonium chloride and the WDC is beeswax, normal $C_{27-33}$

petrolatum, paraffin wax or microcrystalline wax or is from an approximately equal mixture of a petrolatum containing an average of 27 to 33 carbon atoms in the n-alkanes thereof and beeswax.

For the conditioning shampoos of the invention the proportions of synthetic organic detergent, quaternary ammonium salt, WDC and aqueous medium are normally in the ranges of 5 to 25%, 0.2 to 10%, 0.1 to 5% and 65 to 94%, respectively, with such ranges preferably being 8 to 20%, 0.2 to 5%, 0.1 to 3% and 70 to 90%, more preferably being 10 to 18%, 0.4 to 2.0%, 0.1 to 2.0%, and about 80%. For the more preferred conditioning shampoo compositions the anionic synthetic organic detergent is a mixture of at least two different sulfated anionic synthetic organic detergents, of which at least one is an ammonium, sodium or triethanolammonium salt, the quaternary ammonium halide is a higher fatty tri-alkyl lower alkyl ammonium chloride or a higher fatty tri-alkyl di-lower alkyl ammonium chloride wherein the higher alkyl is of 12 to 22 carbon atoms and the lower alkyl is of 1 to 3 carbon atoms, the WDC is selected from the group consisting of alkanes, carboxylic acids, carboxylic acid esters, and alcohols, of alkyl chains of at least 25 carbon atoms, and mixtures thereof. The most preferred compositions comprise 5 to 15% of ammonium and/or sodium lauryl diethoxy sulfate, and the WDC is in a paraffin wax of 25 to 30 carbon atoms and/or in a petrolatum containing an average of 27 to 33 carbon atoms in the n-alkanes thereof, and/or in beeswax.

The wash cycle and rinse cycle softeners for laundry will normally be of percentages of quaternary ammonium salt or equivalent, WDC and aqueous medium (or water) in the ranges of 1 to 20%, 0.1 to 5% and 70 to 97%, respectively, preferably being 1.5 to 10%, 0.1 to 2%, 80 to 97.9%, and more preferably being 1.5 to 7% of di-higher fatty alkyl di-lower alkyl ammonium halide, wherein the higher alkyl is of 12 to 22 carbon atoms and the lower alkyls are of 1 to 3 carbon atoms, 0.1 to 2% of WDC, which is selected from the group consisting of alkanes, carboxylic acids, carboxylic acid esters, and alcohols, of alkyl chains of at least 25 carbon atoms, and mixtures thereof, and 85 to 95% of water. Most preferably, such compositions will comprise 2 to 8% of distearyl dimethyl ammonium chloride, 0.1 to 1% of the WDC, which is in beeswax, petrolatum and/or paraffin wax, and 90 to 95% of water, plus adjuvants, if any.

The softergents will usually be comprised of a detersive proportion of synthetic organic detergent, which normally will be an anionic detergent, nonionic detergent or mixture thereof, 0 to 75% of builder(s) for the detergent, 2 to 20% of fabric softening quaternary ammonium salt or equivalent, 0.1 to 2% of WDC, 0 to 75% of water (with lower water percentages for softergents in particulate form), and the balance, if any of adjuvant(s). Preferably the softergent compositions will comprise 10 to 35% of the synthetic organic detergent, 2 to 15% of quaternary ammonium salt or a quaternary ammonium salt complex, or a mixture thereof, 0.1 to 2% of WDC and 50 to 92% of water, with any balance of adjuvants. More preferably, such softergent can comprise 10 to 25% of a nonionic detergent which is a condensation product of one mole of higher fatty alcohol of 10 to 16 carbon atoms with 3 to 11 moles of ethylene oxide, 2 to 10% of an anionic detergent which is a linear higher alkylbenzene sulfonate wherein the higher alkyl is of 10 to 16 carbon atoms, 7 to 13% of di-higher fatty alkyl di-lower alkyl ammonium halide, wherein the higher alkyl is of 12 to 22 carbon atoms and the lower alkyls are of 1 to 3 carbon atoms, or a citric acid complex thereof, or a mixture thereof, 0.1 to 1% of WDC, which is selected from the group consisting of alkanes, carboxylic acids, carboxylic acid esters, and alcohols, of alkyl chains of at least 25 carbon atoms, and mixtures thereof, and 60 to 75% of water, with any balance being aqueous medium, preferably water. The most preferred softergent compositions comprise 12 to 20% of a nonionic detergent which is a condensation product of one mole of a higher fatty alcohol of 12 to 15 carbon atoms with 5 to 9 moles of ethylene oxide, 3 to 7% of an anionic detergent which is sodium linear alkylbenzene sulfonate wherein the alkyl is of 10 to 14 carbon atoms, 1 to 5% of distearyl dimethyl ammonium chloride, 7 to 11% of a citric acid complex of distearyl methyl amine, 0.1 to 0.5% of the WDC, which is from a petrolatum containing an average length of 27 to 33 carbon atoms in the n-alkanes thereof, and 65 and 70% of water, with any balance of adjuvant(s).

Various procedures for the manufacture of oil-in-water emulsions are well known in the art and such may be employed to make the fiber conditioning compositions of the present invention (when such are in liquid form). However, in order to ensure that the emulsion made will be of improved stability and that the fibre conditioning activity will be maximized and reproducible at such maximum activity it has been found to be desirable to utilize a particular manufacturing process, which is a part of the present invention. Such process comprises forming a melt of the cationic surface active agent component of the fibre conditioning composition and the WDC at an elevated temperature, heating the aqueous medium which is to be the continuous phase of an emulsion, which may contain heat stable hydrophilic components, to a similar temperature, and mixing together the resulting lipophilic and hydrophilic liquids at about the same elevated temperature, whereby the fibre conditioning composition is produced in emulsion form, of improved stability and effectiveness. The temperature to which the cationic surface active agent and the WDC are heated will normally be in the range of 70 to 95 or 100 °C, preferably 70 to 90 °C.and more preferably about 80 °C, e.g., 80 °C. The aqueous medium, preferably containing any stable hydrophilic components of the composition, will be heated to the same temperature, and the lipophilic melt will gradually be admixed into the aqueous medium, so as to form an emulsion, which sometimes will be a microemulsion. The emulsion is cooled gradually to room temperature, usually over a period of five minutes to one hour, with mixing continuing, to avoid any breaking of the emulsion, and any heat sensitive adjuvants are added, with mixing. The room temperature product is a stable liquid in emulsion form. If it separates on lengthy storage at elevated temperature, it may be reformed by shaking. When the fibre conditioning emulsion or other form of the composition is dissolved or dispersed in water as in a wash water or a rinse water, both the cationic surface active agent and the WDC are depositable on fibrous substrates in such water, therby resulting in unexpectedly beneficial and significantly improved conditioning of the fibres treated.

In use, the compositions of this invention are dissolved or dispersed in water at a concentration in the range of 0.01 to 50%. In some instances, as with the hair rinses and shampoos, they may be applied full strength but usually such will be applied in water at a concentration of 10 to 50%, prreferably 20 to 30%. The laundry preparations on the other hand will be used at greater dilutions, such as 0.05 to 2%, e.g. 0.1 to 0.5% and 1%. The normal use temperature is in the range of 10 to 60°C, depending on the type of composition being used. Preferably, for most applications the temperature of the water will be in the range of 10 to 40°C and more preferably such range will be 25 to 40°C, especially for treatment of human hair on the head. The fibrous material to be conditioned, whether human hair, cotton or synthetic fabrics, such as polyester or cotton-polyester blends, will be kept in contact with the aqueous solution or dispersion of the fibre conditioning composition for 1/2 or 1 to 30 minutes, e.g., about 10 minutes, and during that time the cationic surface active agent and the WDC will both deposit on the fibrous substrate asnd will serve to condition it. Often the WDC promotes deposition of the cationic surfactant but sometimes the reverse is also true. In the case of human hair, to which the mentioned active components are applied in conditioning rinse or shampoo form, the important improved conditioning effect noted by evaluation panels is significaantly easier combing, both wet and dry combing, both after application of a conditioning rise of the invention and after shampooing with a conditioning shampoo of the invention. The mechanism of absorption or deposition from the composition containing both cationic surface active fibre conditioning agent and WDC has not been explained but it is clearly more complicated than mere depositions of the composition components, because such components are removed by the fibrous materials from a dilute aqueous solution and are retained on the fibrous substrate despite dilution and subsequent rinsing out of the solution or dispersion of the fibre conditioning composition from the fibrous material. Also, it is an effective conditioning proportion of the active conditioning agents that is left behind on the fibrous material after rinsing. Because sorption of the active conditioning components is better in the absence of significant proportions of builder for the synthetic organic detergent, shampoos, which do not contain such builder, may be of lesser contents of the cationic conditioning agent and the WDC than are desirably incorporated in the softergents of the invention. A very significant advantage of the present invention, however, is that in laundry detergents and shampoos, as well as in conditioning hair rinses and wash cycle and rinse cycle laundry additives, whether for treating human or laundry, the utilizattion of both cationic surface active fibre conditioning agent and WDC results in a significantly improved conditioning action, which thereby permits use of less of the conditioning agent, thereby saving such costs. The products made are of improved stability, too, which is important because the consumer does not favour having to shake liquid products before use to disperse the components thereof and because stable emulsions are much more attractive at the points of sale than are those which have been separated and have to be re-emulsified.

Various advantages of the compositions of the invention and processes have been mentioned above but a specific advantage bears reemphasizing. That is the significant improvement in conditioning action obtained from the combination of cationic surface active fibre conditioning agent and WDC. It has been observed that when human hair is rinsed with compositions of this invention there are significant increases in conditioning effects, such as ease of combing, compared to effects obtained after using compositions which are identical except for the absence of the WDC. Furthermore, the conditioning obtainable is comparable with that of commercial conditioning compositions based on cationic surface active conditioners but containing more of such compounds. The hair conditioning shampoos of the present invention, although containing more active conditioning agents than the rinses, may contain less than commercial hair conditioning shampoos and yet will often be more effective than such commercial products. What is even more surprising, and is considered to be a breakthrough in the art, is that the shampoos of the present invention can be as effective as commercial conditioning rinses. To be able to obtain from a shampoo conditioning like that of a conditioning rinse has heretofore always been considered unattainable. Similar types of improved conditioning effects are noted for the corresponding wash and rinse cycle conditioners and softergents of the invention.

The preferred compositions of this invention are in liquid form and preferably are in stable emulsion or micro-emulsion form. However, the compositions of the present invention, such as the softergents, can be converted to solid or particulate solid form by replacing a substantial proportion or all of the aqueous medium with a solid carrier or solid adjuvants, such as bentonite, sodium sulphate, zeolites, sodium tripolyphosphate, sodium carbonate and other builders. Thus, for example the water content of a liquuid softergent may be replaced by builder or carrier, so that such is up to 75% or more of the composition, and the physical form of the product can be changed accordingly.

The following examples illustrate but do not limit this invention. Unless otherwise indicated all parts are by weight in the examples, in the specification and in the appended claims, and all temperatures are in °C.

| EXAMPLES 1A to 1C | | | |
|---|---|---|---|
| Component | Percent (by weight) | | |
| | 1A | 1B | 1C |
| Cetyl trimethyl ammonium chloride | 1.0 | 1.0 | 1.0 |
| *Petrolatum (Fonoline) (Registered Trade Mark) | - | - | 0.5 |
| Beeswax, natural yellow | - | 0.5 | - |
| Hydroxyethyl cellulose (thickener) | 1.0 | 1.0 | 1.0 |
| Cetyl alcohol | 2.0 | 2.0 | 2.0 |
| Sodium borate | - | 0.07 | - |
| Deionized water | 96.0 | 95.43 | 95.5 |
| | 100.0 | 100.0 | 100.0 |

* contains 60% n-alkanes averaging in the range of 27 to 33 carbon atoms, with distribution curve peak in such range.

Analysis of natural yellow beeswax indicates it to typically contain 23% myricyl palmitate, 2% lacceryl palmitate, 12% myricyl cerotate, and 2% myricyl hypogaete, i.e. 49% of esters of wax acids; and 0.3% pentacosane, 0.3% heptacosane, 1-3% nonacosane, 8-9% hentriacosane and 2.5% of melene, i.e. 12.1 to 15.1% of $C_{25+}$ hydrocarbons.

| EXAMPLES 1D to 1G | | | | |
|---|---|---|---|---|
| Component | Percent (by weight) | | | |
| | 1D | 1E | 1F | 1G |
| Cetyl trimethyl ammonium chloride | 1.0 | 1.0 | 1.0 | 1.0 |
| *Petrolatum (Fonoline) (Registered Trademark) | 0.25 | - | 0.5 | 0.25 |
| Beeswax, natural yellow | 0.25 | 0.5 | - | - |
| Hydroxyethyl Cellulose (thickener) | 1.0 | 1.0 | 1.0 | 1.0 |
| Cetyl alcohol | 2.0 | 2.0 | 2.0 | 1.0 |
| Sodium borate | 0.04 | 0.07 | - | - |
| Deionized water | 95.46 | 95.43 | 95.5 | 97.25 |
| | 100.0 | 100.0 | 100.0 | 100.0 |

*contains 60% n-alkanes averaging in the range of 27 to 33 carbon atoms, with distribution curve peak in such range.

Hair conditioning rinses of the above formulas were made by melting together the lipophilic components (cetyl trimethyl ammonium chloride, petrolatum, beeswax and cetyl alcohol) at 80°C. and emulsifying the melt into the balance of the formula in the aqueous medium, which is also at 80°C., after which the emulsion formed was cooled to room temperature over about 20 minutes for each formula. Standards tresses of human hair (3 grams per tress) were treated with one gram each of the conditioning rinses for one minute each, after which they were rinsed and subjected to wet combing. A panel of evaluators rated ease of wet combing on a scale of 1 to 5, from difficult to easy and 12 ratings were made for each tress, with average ratings being calculated for each. Subsequently, in separate tests, after rinsing of the tresses they were dried by blow drying and were combed. Using the same scale, the ease of combing was again evaluated by the panel, and averages were calculated. The following table summarizes such averages.

TABLE 1

| Types of combing | Averages (ease of combing) | | | | | | |
|---|---|---|---|---|---|---|---|
| | A | B | C | D | E | F | G |
| Wet | 2.52 | 4.50 | 4.25 | 5.15 | 4.25 | 4.20 | 4.10 |
| Dry | 2.98 | 4.75 | 4.21 | 4.25 | 4.52 | 4.22 | 4.17 |

From the resuts of these experiments it is apparent that the presences of the WDC's in the compositions of the present invention substantially increased the ease of combing, which is considered to be closely related to overall manageability and conditioning, of human hair tresses treated with the compositions of the present invention, compared to a control comosition (1A) which did not contain such a WDC. In the experiments reported the best results (best conditioning) were obtained from Experiment 1B, in which the sole WDC is beeswax, but the formula of Experiment 1D yields satisfactory results and is economically advantageous, compared to that of 1B. In vivo tests (actual use tests on "living" hair) verify such results, as do other in vitro tests wherein the tresses are contacted with diluted rinse emulsion (diluted 1:1, 1:2 and 1:3, composition:water).

In similar tests when a mineral oil was employed in place of the WDC's measurably poorer conditioning resulted and sometimes the tresses were noticeably oily, which is usually objectionable. The tresses of Formulas 1A-1G were not oily after treatment. When carnauba wax is employed in place of the WDC's the hair conditioning effect is inferior to the effects of the compositions of the present invention, due to its aromatic compounds' content, but microcrystalline wax is operative.

Although at the present time the costs of such cationic surfactants are prohibitive, by employing heptacosyl trimethyl ammonium chloride or hentriacosyl trimethyl ammonium chloride at 1% or 0.5% concentration in place of cetyl trimethyl ammonium chloride in the present formulas, without any of the named WDC's, those cationic surfactants will also act as WDC's, and the hair conditioning effects will be improved, as in the reported experimental formulas.

In other variations of the formulas of this example the cetyl trimethyl ammonium chloride is replaced by cetyl trimethyl ammonium bromide or with lauryl trimethyl ammonium chloride, the cetyl alcohol is replaced by myristyl alcohol and the petrolatum is replaced by a $C_{25}$ paraffin, and comparable similar improved results are obtained. Also, the proportion of cetyl alcohol to cetyl trimethyl ammonium chloride may be varied in the range of 3:1 to 1:1 from the 2:1 of the examples, and the desired lubricating effect of the cetyl alcohol will still be obtained.

| EXAMPLES 2A to 2C | | | |
|---|---|---|---|
| Components | Percent (by weight) | | |
| | 2A | 2B | 2C |
| Ammonium lauryl sulphate | 7.5 | 12.5 | 12.5 |
| Ammonium laureth sulphate (2EtO) | 7.5 | - | |
| Sodium laureth sulphate (2EtO) | - | 2.5 | 1.5 |
| Lauramide MEA | 1.5 | 2.0 | 2.0 |
| Cetyl alcohol | 1.0 | - | - |
| Stearyl stearate | - | 0.35 | 0.35 |
| Ethylene glycol distearate | 0.5 | 0.75 | 0.75 |
| Propylene glycol | 1.0 | 0.5 | 0.5 |
| Tricetyl methyl ammonium chloride | 1.0 | 0.5 | 0.5 |
| *Petrolatum | - | 0.5 | 0.5 |
| Beeswax (natural yellow) | - | 0.25 | 0.2 |
| Xanthan gum | - | - | - |
| Sodium chloride | 1.0 | 1.5 | 1.5 |
| Preservative (Germaben II) | 0.5 | 0.4 | 0.5 |
| Perfume | 0.5 | 0.5 | 0.4 |
| Deionized water | 78.0 | 77.75 | 77.8 |
| | 100.0 | 100.0 | 100.0 |

| EXAMPLES 2D to 2G | | | | |
|---|---|---|---|---|
| Components | Percent (by weight) | | | |
| | 2D | 2E | 2F | 2G |
| Ammonium lauryl sulphate | 12.5 | 7.5 | 7.5 | 15.0 |
| Ammonium laureth sulphate (2EtO) | - | 7.5 | 7.5 | 3.0 |
| Sodium laureth sulphate (2EtO) | 2.5 | - | - | - |
| Lauramide MEA | 2.0 | 0.75 | 1.5 | 2.0 |
| Cetyl alcohol | - | 0.5 | 2.0 | 0.5 |
| Stearyl stearate | 0.35 | - | - | - |
| Ethylene glycol distearate | 0.75 | 0.5 | 0.5 | - |
| Propylene glycol | 0.5 | 0.5 | 1.0 | - |
| Tricetyl methyl ammonium chloride | 0.5 | 0.5 | 1.0 | 0.5 |
| *Petrolatum | 0.5 | 0.25 | - | - |
| Beeswax (natural yellow) | 0.15 | 0.25 | 0.4 | 2.5 |
| Xanthan gum | - | - | - | 0.3 |
| Sodium chloride | 1.5 | 1.0 | 1.0 | - |
| Preservative (Germaben II) | 0.5 | 0.5 | 0.5 | 0.5 |
| Perfume | 0.4 | 0.4 | 0.5 | 0.5 |
| Deionized water | 77.85 | 79.85 | 76.6 | 75.2 |
| | 100.0 | 100.0 | 100.0 | 100.0 |

*contains 60% n-alkanes averaging in the range of 27 to 33 carbon atoms, with distribution curve peak in such range.

The control emulsion (A) and the emulsions of the present invention (B-G) were all made by the method described in Example 1 and were tested in the same manner. In addition, the evaluators listed their general impressions as to the conditioning of the hair (included in which were considerations of manageability, appearance and feel), as well as wet and dry combing evaluations. Such ratings are given in Table 2 below.

TABLE 2

| Evaluation | Average | | | | | | |
|---|---|---|---|---|---|---|---|
| | A | B | C | D | E | F | G |
| Conditioning | None | *Exc | Exc | Exc | Good | Exc | Exc |
| Wet combing ease | 1.0 | 4.0 | 3.5 | 3.3 | 3.0 | 3.0 | 5.0 |
| Dry combing ease | 1.0 | 4.0 | 3.5 | 3.3 | 3.0 | 3.0 | 5.0 |

*Excellent

From the data presented above it is evident that significant improvement in the conditioning of human hair result from the presence in shampoo formulations of small proportions of the WDC's of this invention together with cationic surfactant hair conditioning compound (quatenary ammonium halide), and such improved conditioning is evidently due to the presence of the WDC because control Formula 2A which contains no WDC, but does contain the cationic conditioning agent, is very much inferior in conditioning effect to compositions 2B-G, all of which are within the present invention. The results described are verified by in vivo tests, which are ordinary shampooings of hair on the head with the described compositions.

In variations of the formulas tested there may be substituted for the described detergent systems those wherein the active detergent components are any of triethanolammonium lauryl sulphate, ammonium olefin sulphonates, ammonium $C_{12-18}$ paraffin sulphonates, or mixtures therof, and nonionic and amphoteric detergents may also be present in such compositions. The cationic surfactant conditioning agent may also be varied, as in Example 1, the WDC may be obtined from other materials, including montan and candelilla waxes, synthetic beeswax and Japan wax, and microcrystalline waxes may be incorporated in the compositions, too. Similarly, the WDC's may be pure compounds, such as hydrocarbons, alcohols, carboxylic acids and carboxylic esters, or of fractions of sources of WDC's, such as beeswax, and may be alcohols, amines, amine salts and quaternary ammonium salts of such hydrocarbons, and amides of carboxylic acids, and the shampoo compositions resulting will also be of improved hair conditioning activities. The WDC's may be those available from certain petrolatums, paraffins and beeswax. The purified components of beeswax, include nonacosane, melissic acid and myricyl cerotate, and n-hentriacontanyl trimethyl ammonium chloride, nonacosyl amine and melissamide, may be considered as derivatives of such purified components. In a further variation of the shampoo formulas a relatively small percentage of nonionic detergent, such a higher fatty alcohol polyethoxylate, like Neodol (Registered Trade Mark) 25-3, 23-6 or 25-7 may be present, in addition to the anionic detergent (to improve removal of oil and previously deposited conditioning agents from hair and scalp). Normally from 1 to 10% of such nonionic detergent may be present, preferably 2 to 5%, e.g. about 3%.

| EXAMPLES 3A to 3C | | | |
|---|---|---|---|
| Component | Percent (by weight) | | |
| | A | B | C |
| Distearyl dimethyl ammonium chloride | 6.0 | 6.0 | 3.0 |
| **Amine citrate complex | - | - | 3.0 |
| Beeswax (yellow) | - | 0.5 | 0.5 |
| *Petrolatum | - | 0.1 | 0.1 |
| Sodium citrate | 0.05 | 0.05 | - |
| Propylene glycol | 0.1 | 0.1 | 0.1 |
| Deionized water | 93.85 | 93.25 | 93.3 |
| | 100.0 | 100.0 | 100.0 |

* contains 60% n-alkanes averaging in the range of 27 to 33 carbon atoms, with its distribution curve peak in such range

** pre-made complex obtained by mixing distearyl methyl amine and citric acid in a molar ratio of 1:1

The wash cycle and rinse cycle fabric conditioning compositions of this example, which are useful for the treatmeent of laundry, usually in an automatic washing machine, are made by following the procedure described in Examplel, with the lipophilic materials being melted together and being admixed with the hydrophilic components in the aqueous medium, and being cooled to room temperature.

The control (3A) and the compositions of the present invention (3B and 3C) are each tested in wash cycle and rinse cycle applications, using different commercial detergents at the concentrations in the wash waters that were suggested by the manufacturers thereof. In the wash cycle tests the wash water is of a hardness of about 125 p.p.m., as $CaCO_3$ and is at a temperature of 32° C, the fabric load is large, the wash time is fourteen minutes and the rinse is cold. The commercial detergents to which the wash cycle additive are added are TIDE (Registered Trademark) powder (with phosphate builder), FRESH START (Registered Trade Mark) liquid TIDE, and liquid WISK (Registered Trade Mark, and the concentrations of the wash cycle additive in the wash water are varied so that from about 4 to 9% of conditioning components are present therein, based on the detergent composition employed (or 75 to 150 g. of conditioning composition per 68 litres of wash water, which is 0.1 to 0.2%). The test items, which are mixed in with an ordinary fabric ballast load of five pounds of mixed fabric types, include cotton, nylon tricot, Dacron (Registered Trade Mark) single knit, Dacron double knit, 65/35 Dacron/cotton blends and Banlon (Registered Trade Mark) swatches (for static pick-iup evaluations), plus terrycloth hand towels, for softness evaluations. The washed materials are dried for 60 minutes, using the heavy, high setting of an automatic laundry dryer, and the entire washing and drying operations are repeated twice, after which the test fabrics are evaluated for static cling (synthetics) and softness (terrycloth towels.) The evaluation for static cling is conducted immediately after the dryer has been shut off after the third drying. The humidity in the test room is in the range of 44 to 56% R.H. for both static and softness tests. The terrycloth towels are hung on a clothes rack after being removed from the dryer and are allowed to remain there for about twelve hours before being evaluated for softness.

At all concentrations of the active conditioning components that were tested and in washing the test fabrics with all of the detergent compositions in wash water containing the 3B and 3C wash cycle additives, less static cling was noted that when composition 3A was employed. The differences in cling are apparent to even a casual observer. With respect to fabric softness, the results are similarly favorable to the compositions of the present invention, both when evaluations are conducted by an expert of long experience in such techniques, and when observations are made by unskilled observers.

A very important result that was observed is that employment of the compositions of the present invention as wash cycle softeners results in even better fabric softening and less static cling than that which results when a commercial rinse cycle softener (Downy) (Registered Trade Mark) in an amount of equal active conditioning ingredient content, is employed as a wash cycle additive.

In rinse cycle applications, utilizing the same procedure, except for utilizing only one washing operation and one drying, the same types of results are obtained.

A theory that has been advanced to explain the superiority of the detergent compositions of the present invention over controls and over the commercial fabric softener in wash cycle applications is that applicants' WDC acts to "protect" the cationic surfactant from reaction with the anionic compounds in the wash water, allowing it to exert its full fabric conditioning capabilities. Such theory is supported by measurements of specific conductances and Zeta potentials of the experimental and control compositions, and turbidity measurements of the compositions of the present invention and controls in wash waters containing commercial anionic detergent compositions, but is not relied on herein.

In variations of the examples given the various components, other than cationic surface active conditioning agent and WDC, may be omitted, and the results reported will be essentially the same. Also, the different cationic components and sources of WDC's previously mentioned in the specification and in the other working examples may be substituted for those of Example 3, and similar significant differences in fabric softening and in antistatic action, in favour of the compositions of the present invention will be obtained. Thus, for example, mono-higher alkyl tri-lower alkyl ammonium halides and tri-higher alkyl mono-lower alkyl ammonium haides may be employed, as may be others of the WDC's previously mentioned.

| EXAMPLES 4A to 4B | | |
|---|---|---|
| Component | Percent (by weight) | |
| | A | B |
| Sodium linear dodecylbenzene sulphonate | 5.0 | 5.0 |
| Sodium sulphate (accompanying anionic detergent) | 1.7 | 1.7 |
| ***Neodol 25-7 | 15.0 | 15.0 |
| Distearyl dimethyl ammonium chloride | 6.0 | 6.0 |
| *Petrolatum | - | 0.5 |
| Citric acid (50% aqueous solution) | 9.0 | 9.0 |
| Triethanolamine | 0.3 | 0.3 |
| Formalin | 0.2 | 0.2 |
| Fluorescent brightener (Tinapol) (Registered Trademark 5BM, Extra conc.) | 0.2 | 0.2 |
| Colorant (0.5% aqueous solution) | 1.0 | 1.0 |
| Perfume | 0.4 | 0.4 |
| Deionized water | 61.2 | 60.7 |
| | 100.0 | 100.0 |

*contains 60% n-alkanes having in the range of 27 to 33 carbon atoms, with distribution curve peak in such range.
*** Nonionic detergent, which is a condensation product of a mole of fatty alcohol of 12-15 carbon atoms with seven moles of ethylene oxide.

A control softergent composition (4A) and the composition of the present invention (4B) are both employed to wash both cotton and synthetic test fabrics, following the procedure described in Example 3, with only a single washing and drying, and the fabrics are subsequently evaluated for static cling and softness by the methods described in Example 3. The results obtained are also like those reported in Example 3 for wash cycle fabric conditioning compositions, which is as expected because the present softergent composition produces substantially the same type of wash water as results from addition of the corresponding wash cycle conditioning composition to a wash water containing detergent composition. As in Example 3, when multiple washings and dryings of the test fabrics are practiced the improved conditioning effects, which are in fovour of the present invention are enhanced.

In modifications of the composition formula the detergent component may be all anionic or all nonionic, and the anionic and nonionic detergents may be replaced with others previously described in these examples and in the foregoing specification, the distearyl dimethl ammonium chloride may be replaced by other quaternary ammonium salts, such as those previously referred, or by other amine and quaternary ammonium complexes, such as those previously mentioned. Also, the WDC may be replaced by other WDC's of the types that had been earlier described. Such a change or such a plurality of changes still results in compositions of the present invention which will be of improved fabric conditioning properties, including softening and/or antistatic properties, compared to control compositions which do not contain the required WDC. Another advantage of the invention is that the various WDC's are all safe to employ in the described proportions (many having already been accepted or approved for oral ingestion) and so do not require toxicity testing and governmental clearances.

EXAMPLE 5

In the formulas and compositions described in the previous examples variations of the components may be made by replacement of one or more of them with others described in the specification as equivalents or substitutes. The proportions of components may be varied, usually 10%, 20% and 50%, providing that they remain within the ranges recited in the specification. Liquid compositions may be converted to particulate solids by replacement of water with particulate carrier materials, such as builders and fillers. In some instances it may be desirable separately to add to the wash or rinse waters the fused mixture of cationic surfactant fibre conditoning agent and WDC, preferably in liquid form either melted or in an appropriate organic solvent, such as isopropanol, or mixed in with a powdered carrier. The individual cationic surfactant fibre conditioning agent and WDC may also be added to the wash waters or rinse waters but, as had previously been indicated, the desirable fibre conditioning effects will not be obtained from such a mere mixture to as great an extent as when such components are first fused together. Furthermore, it is highly preferable that they should be in emulstion form when charged to the wash and rinse waters. In an extension of the invention, on which work is now pending, the various WDC's, including those which are quaternary ammonium salts, are employed to promote deposition on or sorption by substrates, such as hair or laundry fibres, of relatively insoluble materials, such as fluorescent brighteners, colorants, perfumes, soil release promoting polymers, fungicides, bactericides, insect repellents, soil repellents and crease-proofing chemicals, which may be incorporated in appropriate rinses, additives and detergent compositions.

One preferred form of the invention provides a composition useful as a conditioning rinse for human hair after shampooing, characterized in that it comprises 0.2 to 10%, e.g. 0.2 to 5%, of cationic surface active fibre conditioning agent, 0.1 to 5%, e.g. 0.1 to 2%, of water dispersible compound, 75 to 99%, e.g. 85 to 98%, of aqueous medium, and the balance if any, of adjuvant(s).

Preferably it comprises 0.3 to 2% of higher fatty alkyl tri-lower alkyl ammonium halide, di-higher fatty alkyl di-lower alkyl ammonium halide, or tri-higher fatty alkyl lower alkyl ammonium halide wherein the higher alkyl is of 12 to 22 carbon atoms and the lower alkyls are of 1 to 3 carbon atoms, 0.1 to 1% of water dispersible compound which is selected from the group consisting of alkanes, carboxylic acids, carboxylic acid esters, and alcohols, of alkyl chains of at least 25 carbon atoms, and mixtures thereof, 90 to 97% of water, and the balance, if any, of adjuvant(s).

More preferably it comprises 0.4 to 0.2% of cetyl trimethyl ammonium chloride, dicetyl dimethyl ammonium chloride, or distearyl dimethyl ammonium chloride, and 0.1 to 0.5% of the water dispersible compound, which is in an approximately equal mixture of a petrolatum containing an average of 27 to 33 carbon atoms in the n-alkanes thereof, and in beeswax, 95 to 97% of water, and the balance, if any, of adjuvant(s).

Another preferred form of the invention provides a composition useful as a conditioning shampoo for human hair characterized in that it comprises 5 to 25%, e.g. 8 to 20%, of synthetic organic detergent, e.g. anionic synthetic organic detergent,

Another preferred form of the invention provides a composition useful as a conditioning shampoo for human hair characterized in that it comprises 5 to 25%, e.g. 8 to 20%, of synthetic organic detergent, e.g. anionic synthetic organic detergent, 0.2 to 10%, e.g. 0.2 to 5%, of cationic surface active fibre conditioning agent, 0.1 to 5%, e.g. 0.1 to 2%, of water dispersible compound, 65 to 94%, e.g. 70 to 90%, of aqueous medium, and the balance, if any, of adjuvant(s).

Preferably it comprises 10 to 18% of a mixture of at least two different sulphated anionic synthetic organic detergents, of which at least one is of an ammonium, sodium or triethanolamine salt, 0.3 to 2% of tri-higher fatty alkyl mono-lower alkyl ammonium chloride, wherein the higher alkyl is of 12 to 22 carbon atoms and the lower alkyl is of 1 to 3 carbon atoms, 0.1 to 1.5% of cationic surface active fibre conditioning agent, which is selected from the group consisting of alkanes, carboxylic acids, carboxylic acid esters, alcohols, amides, amines, amine salts, and quaternary ammonium salts of alkyl chains of at least 25 carbon atoms, and mixtures thereof, 75 to 65% of water, and the balance, if any, of adjuvant(s).

More preferably it comprises 5 to 15% of ammonium and/or sodium lauryl diethoxy sulphate, 1 to 10% of ammonium and/or sodium lauryl diethoxy sulphate, 0.4 to 2.0% of tri-cetyl methylammonium chloride, 0.1 to 2.0% of the water dispersible compound, which is in a petrolatum containing an average of 27 to 33 carbon atoms in the n-alkanes thereof, a paraffin wax containing an average of 25 to 30 carbon atoms and/or in beeswax, about 80% of water, and the balance, if any, of adjuvant(s).

Another preferred form of the invention provides a composition useful as a wash cycle or rinse cycle fabric softener for laundry characterized in that it comprises 1 to 20% of a fabric softening quaternary ammonium salt, cationic amine, quaternary ammonium salt complex with anionic surfactant, or complex of cationic amine, or any mixture thereof, 0.2 to 5% of water dispersible compound, 70 to 97.9% of aqueous medium, and the balance, if any, of adjuvant(s).

Preferably it comprises 1.5 to 10% of fabric softening quaternary ammonium salt, 0.1 to 2% of water dispersible compound, 70 to 97.9% of water, and the balance, if any, of adjuvant(s).

More preferably it comprises 1.5 to 7% of di-higher fatty alkyl di-lower alkyl ammonium halide, wherein the higher alkyls are of 12 to 22 carbon atoms and the lower alkyls are of 1 to 3 carbon atoms, 0.1 to 2% of water dispersible compound, which is selected from the group consisting of alkanes, carboxylic acids, carboxylic acid esters, amines or amine salts, amides, quaternary ammonium salts, and alcohols, of alkyl chains of at least 25 carbon atoms, and mixtures thereof, 85 to 95% of water, and the balance, if any of adjuvant(s).

15

More specifically it comprises 2 to 8% of distearyl dimethyl ammonium chloride, 0.1 to 1% of the water dispersible compound which is beeswax, petrolatum and/or paraffin wax, 90 to 95% of water and the balance, if any, of adjuvant(s).

Another preferred form of the invention provides a composition useful as a softergent to wash laundry and soften it, characterized in that it comprises a detersive proportion of a synthetic organic detergent, 0 to 75% of builder(s) for the detergent, 2 to 20% of a fabric softening quaternary ammonium salt, cationic amine, quaternary ammonium salt complex with anionic surfactant, complex of cationic amine, or any mixture thereof, 0.1 to 2% of water dispersible compound, 0 to 75% of water, and the balance, if any, of adjuvant(s).

Preferably it comprises 10 to 35% of a synthetic organic detergent selected from the group consisting of anionic and non ionic detergents and mixtures thereof, 2 to 15% of a quaternary ammonium salt or a quaternary ammonium salt complex, or a mixture thereof, 0.1 to 2% of water dispersible compound, 50 to 92% of water, and the balance, if any, of adjuvant(s).

More preferably it comprises 10 to 25% of a nonionic detergent which is a condensation product of one mole of a higher fatty alcohol of 10 to 16 carbon atoms with 3 to 11 moles of ethylene oxide, 2 to 10% of an anionic detergent which is a linear higher alkylbenzene sulfonate, wherein the higher alkyl is of 10 to 16 carbon atoms and the lower alkyls are of 1 to 3 carbon atoms, or a citric acid complex thereof, or a mixture thereof, 0.1 to 1% of water dispersible compound which is selected from the group consisting of alkanes, carboxylic acids, carboxylic acid esters, and alcohols, of alkyl chains of at least 25 carbon atoms, and mixtures thereof, 60 to 75% of water, and the balance, if any of adjuvant(s).

More specifically it comprises 12 to 20% of a nonionic detergent which is a condensation product of one mole of a higher fatty alcohol of 12 to 15 carbon atoms with 5 to 9 moles of ethylene oxide, 3 to 7% of an anionic detergent which is sodium linear alkylbenzene sulphonate wherein the alkyl is of 10 to 14 carbon atoms, 1 to 5% of distearyl dimethyl ammonium chloride, 7 to 11% of a citric acid complex of distearyl methyl amine, 0.1 to 0.5% of the water dispersible compound which is in a petrolatum containing an average chain length of 27 to 33 carbon atoms in the n-alkanes thereof, 65 to 70% of water, and the balance, if any, of adjuvant(s).

In another broad aspect the invention extends to a fibre conditioning composition containing a cationic surface active fibre conditioning agent, for example a quaternary ammonium compound, in which at least one of the alkyl substituents contains at least 25 carbon atoms.

The invention has been described with respect to working examples, illustrations and embodiments thereof but is not to be considered as limited to these because it is evident that one of skill in the art, with the present specification before him/her, will be able to utilize substitutes and equivalents without departing from the invention.

## Claims

1. A fibre conditioning composition which comprises a cationic surface active fibre conditioning agent and a water dispersible compound which contains in its formula a long chain hydrocarbyl group of at least 25 carbon atoms, the proportion of cationic surface active fibre conditioning agent to water dispersible compound being such that the deposition of at least one of them on to a fibrous substrate to be conditioned thereby, by application to the substrate of an aqueous medium containing the said composition, is improved.

2. A composition as claimed in claim 1 in which the proportion of cationic surface active fibre conditioning agent to long chain hydrocarbyl-containing compound is in the range of 1:20 to 20:1, in which proportion the presence of both such materials in the composition improves the deposition of at least one of them on a fibrous substrate to be conditioned by application to it of an aqueous medium containing such composition.

3. A composition as claimed in claim 1 or claim 2 characterised in that cationic surface active fibre conditioning agent is a quaternary ammonium salt and the water dispersible compound is a hydrocarbon, an acid, an ester, an amide, an amine, an amine salt, a quaternary ammonium salt, or an alcohol or any mixture of any thereof, the composition is aqueous, the proportion of such quaternary ammonium salt to such water dispersible compound is in the range of 0.5:1 to 20:1, and the deposition of at least one of the mentioned compounds on a substrate to be conditioned is on human hair or laundry.

4. A composition as claimed in claim 1, 2 or 3 characterized in that the cationic surface active fibre conditioning agent and water dispersible compound are in an aqueous emulsion in which the proportion of cationic surface active fibre conditioning agent is in the range of 0.1 to 10%.

5. A process for manufacturing a fibre conditioning composition as claimed in any one of claims 1 to 5 in stable emulsion form characterized in that it comprises forming a melt of the cationic surface active fibre conditioning agent and the water dispersible compound at a temperature in the range of 70 to 90°C, with other heat stable lipophilic components of the fibre conditioning composition, if any, heating an aqueous medium, which may contain heat stable hydrophilic components of the fibre conditioning composition, to a termperature in the range of 70 to 90°C, mixing together the resulting liquids at about the same temperature to manufacture the fibre conditioning composition in stable emulsion form.

6. A process as claimed in claim 5 characterized in that the lipophilic liquid and the hydrophilic liquid components of the fibre conditioning liquid composition are mixed together at a temperature of about 80°C. and cooled to room temperature, with stirring, after which any heat sensitive components are admixed therewith.

7. A process for treating a fibrous material to condition it characterized in that it comprise applying to such fibrous material a fibre conditioning composition as claimed in any one of claims 1 to 4 or the product of claims 5 or 6, in dispersion in water at a concentration of 0.01 to 50%, for 1 to 30 minutes at a temperature in the range of 10 to 40°C and rinsing the solution or dispersion of the fibre conditioning composition from the fibrous material, which leaves behind on the fibrous material at least some of either the cationic surface active fibre conditioning agent or the water dispersible compound component of the fibre conditioning composition or both.

8. A process as claimed in claim 7 characterized in that the fibre conditioning composition is a rinse composition for human hair which comprises 0.2 to 10% of a cationic surface active fibre conditioning agent, 0.1 to 5% of water dispersible compound, 75 to 99% of aqueous medium, and the balance if any, of adjuvant(s), and the rinse composition is in dispersion in water at a concentration of 10 to 50%.

9. A process as claimed in claim 7 characterized in that the fibre conditioning composition is a conditioning shampoo for human hair which comprises 5 to 25% of synthetic organic detergent, 0.2 to 10% of a cationic surface active fibre conditioning agent, 0.1 to 5% of water dispersible compound, 65 to 94% of aqueous medium, and the balance, if any, of adjuvant(s), and the shampoo is in solution or dispersion in water at a concentration of 10 to 50%.

10. A process as claimed in claim 7 characterized in that the fibre conditioning composition is a wash cycle or rinse cycle fabric softener for laundry which comprises 2 to 20% of a cationic surface active fibre conditioning agent, optionally a fabric softening quaternary ammonium salt, cationic amine, quaternary ammonium salt complex with anionic surfactant, or complex of cationic amine, or any mixture thereof, 0.2 to 5% of water dispersible compound, 70 to 97% of aqueous medium, and the balance, if any, of adjuvant(s), and the wash cycle or rinse cycle fabric softener is in solution or dispersion in water at a concentration of 0.05 to 2%.

11. A process as claimed in claim 7 characterized in that the fibre conditioning composition is a softergent which comprises a detersive proportion of a synthetic organic detergent, 0 to 75% of a builder for the detergent, 2 to 20% of a cationic surface active fibre conditioning agent, optionally a fabric softening quaternary ammonium salt, cationic amine, quaternary ammonium salt complex with anionic surfactant, complex of cationic amine, or any mixture thereof, 0.1 to 0.5% of water dispersible compound, 0 to 75% of water, and the balance, if any, of adjuvant(s), and the softergent is in solution or dispersion in wash water at a concentration of 0.1 to 2%.